# EUROPEAN PATENT APPLICATION

(11) **EP 4 606 443 A1**
(43) Date of publication of application: **27.08.2025**
(21) Application number: 23879510.8
(22) Date of filing: 11.09.2023
(51) Int. Cl.: A63B 71/06, A61B 5/02, A61B 5/0245, A61B 5/145, A61B 5/22, A63B 69/00

(54) **MAXIMAL EXERCISE INTENSITY ESTIMATION METHOD, TRAINING METHOD, EXERCISE INSTRUCTION DEVICE, AND MAXIMAL EXERCISE INTENSITY ESTIMATION SYSTEM**

(30) Priority: 20.10.2022 JP 2022168216
(71) Applicant: Fancl Corporation, Kanagawa 231-8528 (JP)
(72) Inventor: ABE, Masatsugu, Yokohama-shi, Kanagawa 244-0806 (JP); HIROISHI, Ryoichi, Yokohama-shi, Kanagawa 244-0806 (JP); MIYAKE, Ai, Yokohama-shi, Kanagawa 244-0806 (JP)
(74) Representative: Hasegawa, Kan
(86) International application number: PCT/JP2023/032986
(87) International publication number: WO 2024/084860

(57) **Abstract**

An object is to provide a method, and a system, for estimating maximal exercise intensity, a training method whereby exercise is performed using the maximal exercise intensity estimated by the estimation method as a guideline, as well as an exercise instruction device capable of instructing, using an individual's maximal exercise intensity as a guideline, an exercise intensity that represents the person's maximal exercise intensity. As a solution, a method for estimating maximal exercise intensity, an estimation system capable of implementing this estimation method, as well as a training method, and an exercise instruction device, utilizing this estimation method, are provided, wherein such estimation method comprises: a step to give a ramping load to a subject to obtain a measured value of blood oxygen concentration (SpO₂) at each different workload over a range that covers at least a part of 90 to 100%, while measuring the pulse rate simultaneously with the SpO₂; and a step to determine an inflection point at which the behavior of SpO₂/pulse rate changes as the workload increases beyond an optimal exercise intensity; wherein the exercise intensity at the inflection point is estimated as a maximal exercise intensity for the subject.

## Description

### Technical Field

The present invention relates to a maximal exercise intensity estimation method, a training method, an exercise instruction device, and a maximal exercise intensity estimation system.

### Background Art

A number of reports have shown that the greater a person's body strength demonstrated by muscle strength, cardiorespiratory capacity, etc., the better the person's health, higher his/her rate of survival, and lower his/her mortality will become (Non-patent Literatures 1, 2). Improving the muscle strength, cardiorespiratory capacity, and other measures of body strength requires exercising at moderate or higher intensity; for example, one cannot improve his/her body strength by continuing exercises that lack intensity. While what moderate intensity exercise means varies from one individual to another, it is known that the anaerobic threshold (AT) at which aerobic exercise switches to anaerobic exercise marks an onset of exercise of moderate intensity for all people.

The AT corresponds to the lactate threshold (LT) at which the lactate concentration in blood starts to rise rapidly due to increased exercise intensity, or to the ventilation threshold (VT) at which the rate of increase in the carbon dioxide in exhaled breath rises markedly due to increased exercise intensity. Traditionally, these thresholds are measured by two methods that are known as the golden standard methods, including a testing method employing breath gas analysis equipment and a method using the blood lactate concentration. Measurement based on the golden standard methods requires the subject to exercise at gradually increasing intensities until his/her maximum exercise intensity is reached, i.e., until just before he/she can no longer move, which puts significant physical and mental burdens on the subject. Furthermore, measuring the blood lactate concentration in a manner producing accurate values requires a blood draw that can be painful, although noninvasive estimation methods have been proposed (Patent Literatures 1, 2, etc.). Also, measuring the carbon dioxide concentration in exhaled breath requires the subject to exercise while breathing through a mouthpiece connected to a breath gas measuring device, which means that an expensive special measuring device must be used in the presence of an expert (Patent Literature 3). Because of the need for the subject to exercise under the supervision and guidance of an instructor until just before he/she can no longer move, along with the need for special equipment and possibly for a blood draw, AT measurement by the golden standard methods could only be performed at hospitals, universities with sports education programs, research institutes, etc.

The applicant of the present application for patent has proposed in Patent Literature 4 a method for estimating an individual's optimal exercise intensity corresponding to the AT by giving a ramping load to obtain a measured value of blood oxygen concentration (SpO₂) taken at each different workload, without requiring the person to exercise until his/her maximum exercise intensity is reached.

Here, one standard of exercise intensity higher than the AT is the respiratory compensation point (RCP) that represents the maximal exercise intensity one can sustain before metabolic acidosis causes the person to hyperventilate severely. While the rate of increase in the carbon dioxide in blood rises markedly at an exercise intensity equal to or above the AT, increasing the exercise intensity further causes the carbon dioxide concentration in blood to also rise concurrently. The RCP refers to the point at which the body starts to actively discharge this carbon dioxide in blood, which had increased concurrently with the increase in exercise intensity, through an increased number of breaths.

It is known that athletes and other individuals wanting to attain exceptionally high levels of motor abilities can efficiently improve their physical abilities by exercising at intensities that are inside a range from the AT to RCP and also correspond to approx. 80 to 95% of the exercise intensity at the RCP, with the RCP being the upper limit.

This RCP, too, is known to rely on a method employing breath gas analysis equipment as a golden standard method, which means that an individual must exercise at gradually increasing intensities until his/her maximum exercise intensity is reached, i.e., until just before he/she can no longer move, thereby putting significant physical and mental burdens on the individual.

### Background Art Literature

### Patent Literature

Patent Literature 1: Japanese Patent Laid-open No. Hei 9-126995
Patent Literature 2: Japanese Patent Laid-open No. 2011-232320
Patent Literature 3: Japanese Patent Laid-open No. 2018-134294
Patent Literature 4: Japanese Patent No. 6990333

### Non-patent Literature

Non-patent Literature 1: Blair SN et al., Physical fitness and all-cause mortality. A prospective study of healthy men and women. JAMA. 1989; 262 (17):2395-2401.
Non-patent Literature 2: Jonathan Myers, Manish Prakash, Victor Froelicher, et al., Exercise Capacity and Mortality among Men Referred for Exercise Testing. Engl J Med 2002; 346:793-801.

### Summary of the Invention

### Problems to Be Solved by the Invention

An object of the present invention is to provide a method, and a system, for estimating an individual's maximal exercise intensity without requiring the person to exercise until his/her maximum exercise intensity is reached, i.e., until just before he/she can no longer move, a training method whereby exercise is performed using the maximal exercise intensity estimated by the estimation method as a guideline, as well as an exercise instruction device, and an exercise instruction system, capable of instructing an exercise intensity using the individual's maximal exercise intensity as a guideline.

### Means for Solving the Problems

The means for achieving the object of the present invention are as follows.
1. A method for estimating maximal exercise intensity characterized in that it comprises:
   a step to give a ramping load to a subject to obtain a measured value of blood oxygen concentration (SpO₂) at each different workload over a range that covers at least a part of 90 to 100%, while measuring the pulse rate simultaneously with the SpO₂; and
   a step to determine an inflection point at which the behavior of SpO₂/pulse rate changes as the workload increases beyond an optimal exercise intensity;
   wherein the exercise intensity at the inflection point is estimated as a maximal exercise intensity for the subject.
2. The method for estimating maximal exercise intensity according to 1, wherein the optimal exercise intensity represents the exercise intensity at the first inflection point at which the behavior of SpO₂/pulse rate changes, or exercise intensity at a starting point of decline at which the measured value of blood oxygen concentration starts to show a declining trend.
3. A training method characterized in that exercise is performed using, as a guideline, the maximal exercise intensity estimated by the estimation method according to 1 or 2.
4. An exercise instruction device characterized in that it comprises:
   a storage means for storing biological information values at the maximal exercise intensity estimated by the estimation method according to 1 or 2;
   a measurement means capable of measuring the biological information values;
   a computation means for calculating an exercise intensity by comparing the biological information values measured by the measurement means against the biological information values at the maximal exercise intensity; and
   an instruction means for instructing the exercise intensity calculated by the computation means.
5. The exercise instruction device according to 4, characterized in that:
   the measurement means is capable of measuring the blood oxygen concentration (SpO₂);
   the instruction means is capable of instructing a workload based on information relating to biological information values from the measurement means; and
   the computation means is capable of calculating an inflection point at which the behavior of SpO₂/pulse rate changes as the workload increases beyond an optimal exercise intensity.
6. The exercise instruction device according to 4, characterized in that it is a wearable terminal.
7. A system for estimating maximal exercise intensity characterized in that it comprises:
   a measurement part that measures the blood oxygen concentration (SpO₂) over a range that covers at least a part of 90 to 100%, while also measuring the pulse rate;
   an instruction part that instructs a workload that will be used as a ramping load; and
   a computation part that calculates an inflection point at which the behavior of SpO₂/pulse rate changes as the workload increases beyond an optimal exercise intensity;
   wherein the exercise intensity at the inflection point is estimated as a maximal exercise intensity.
8. The system for estimating maximal exercise intensity according to 7, wherein the optimal exercise intensity represents the exercise intensity at the first inflection point at which the behavior of SpO₂/pulse rate changes, or exercise intensity at a starting point of decline at which the measured value of blood oxygen concentration starts to show a declining trend.

### Effects of the Invention

According to the present invention, an individual's maximal exercise intensity can be derived in a simple but accurate and economical manner, which can contribute to an efficient improvement of the person's motor abilities. To derive an individual's maximal exercise intensity using the conventional methods, the person must exercise under the supervision and guidance of an instructor until just before he/she can no longer move, which puts significant physical and mental burdens on the person. The present invention can accurately obtain an individual's maximal exercise intensity without requiring the person to exercise at his/her maximum exercise intensity, thereby allowing the individual to know his/her maximal exercise intensity frequently compared to when the conventional methods are used. The present invention allows an individual to obtain his/her maximal exercise intensity frequently compared to when the conventional golden standard methods are used, which is quite revolutionary for professional athletes, top amateur athletes, and others who are required to demonstrate high levels of performance. Additionally, the present invention is useful not only for competitive athletes, but also for health-conscious individuals looking to incorporate exercise habits into their way of life.

### Mode for Carrying Out the Invention

### • Estimation Method and Estimation System

The method for estimating maximal exercise intensity proposed by the present invention is characterized in that it comprises:
a step to give a ramping load to a subject to obtain a measured value of blood oxygen concentration (SpO₂) at each different workload over a range that covers at least a part of 90 to 100%, while measuring the pulse rate simultaneously with the SpO₂; and
a step to determine an inflection point at which the behavior of SpO₂/pulse rate changes as the workload increases beyond an optimal exercise intensity;
wherein the exercise intensity at the inflection point is estimated as a maximal exercise intensity for the subject.

It should be noted that, in this Specification, "SpO₂/pulse rate" indicates a value calculated by dividing the SpO₂ by the pulse rate, while "A to B (A and B are numbers)" indicates a range of numerical values including the values of A and B, or specifically A or greater but no greater than B.

The blood oxygen concentration (SpO₂) is a ratio of binding of the red blood cell hemoglobin and oxygen in arterial blood. The SpO₂ can be measured simply by wearing a measuring device (pulse oximeter) at the fingertip, over the wrist, etc. The method for estimating maximal exercise intensity proposed by the present invention is noninvasive and therefore puts minimal burdens on the subject.

The estimation method proposed by the present invention has a step to give a ramping load to a subject to obtain a measured value of blood oxygen concentration (SpO₂) at each different workload over a range that covers at least a part of 90 to 100%.

The exercise method for giving a ramping load is not specifically limited, and a treadmill, bicycle ergometer, stepper, etc., may be adopted.

It suffices that the measured value of SpO₂ falls within a range that covers at least a part of 90 to 100%, where the measurement band is preferably 2% or greater, or more preferably 3% or greater, or yet more preferably 4% or greater, or even more preferably 5% or greater, such as 89 to 100%, 92 to 100%, 94 to 100%, 95 to 100%, 96 to 100%, 92 to 99%, 94 to 99%, 95 to 99%, etc. A smaller minimal value of the SpO₂ measuring range allows for more accurate estimation of maximal exercise intensity but increases the exercise burdens during measurement. For this reason, preferably a minimal value is set for each subject according to his/her sex, age, exercise habits or lack thereof, etc., and measurement is stopped once the measured value of SpO₂ drops below the set minimal value. In particular, the lower the blood oxygen concentration (SpO₂), the greater the burdens on the subject will become, and therefore its minimal value is preferably 90% or greater, or more preferably 92% or greater, or yet more preferably 94% or greater, or even more preferably 96% or greater.

The SpO₂, etc., can be measured continuously, but since measurement is performed during exercise, the measuring devices may shift and prevent accurate measured values from being obtained. For this reason, preferably measured values obtained from intermittent measurements taken every 0.1 to 5 seconds or so are consolidated into average values or median values over 1 to 30 seconds or so and used as such. Also, since the workload gradually increases under a ramping load, the biological information values that are measured normally change only in one direction-for example, the SpO₂ changes only in the decreasing direction, while the pulse rate changes only in the increasing direction-and this allows for employment of processes that do not use values indicating change in the opposite-to-normal direction, processes that do not use values with a rate of deviation of, for example, 10% or greater from the value measured immediately before or from the average or median of values measured at around 2 to 5 prior measurement points, and so on.

The estimation method proposed by the present invention measures the pulse rate simultaneously with the SpO₂. Furthermore, the estimation method proposed by the present invention allows for measurement of one type, or two or more types, of biological information value(s) including the blood pressure, lactate concentration (in blood, in sweat), carbon dioxide concentration in exhaled breath, etc., besides the SpO₂ and pulse rate.

Under the estimation method proposed by the present invention, preferably measurement is performed in a range of 90% or higher in blood oxygen concentration and also 180 beats/ or lower in pulse rate, and so on, because doing so can reduce the burdens on the subject.

The estimation method proposed by the present invention has a step to determine an inflection point at which the behavior of SpO₂/pulse rate changes as the workload increases beyond an optimal exercise intensity.

As the workload increases, the SpO₂ decreases and the pulse rate increases, and consequently the SpO₂/pulse rate indicates a falling trend. That this SpO₂/pulse rate has, post-optimal exercise intensity, an inflection point at which its slope steepens, and that this inflection point approximates the respiratory compensation point (RCP), are new insights gained by the inventors of the present invention.

The estimation method proposed by the present invention can be implemented by, for example, a system for estimating maximal exercise intensity characterized in that it comprises:
a measurement part that measures the blood oxygen concentration (SpO₂) over a range that covers at least a part of 90 to 100%, while also measuring the pulse rate;
an instruction part that instructs a workload that will be used as a ramping load; and
a computation part that calculates an inflection point at which the behavior of SpO₂/pulse rate changes as the workload increases beyond an optimal exercise intensity;
wherein the exercise intensity at the inflection point is estimated as a maximal exercise intensity.

The estimation system proposed by the present invention may also have, besides the above, a storage part that stores the measured values, etc., a communication part that exchanges data externally, a display part that displays the details of instructions, measured values, etc., and so on. Also, the storage part and computation part may be at least partially comprised of a cloud-based system whose processing takes place on an external server with which it communicates through the communication part. Furthermore, the estimation system proposed by the present invention may be, for example, a smartphone, smart watch, smart glasses, earphone, or other wearable device that is made capable of implementing the aforementioned means by installing therein an application having a function to estimate maximal exercise intensity. The estimation system proposed by the present invention may be constituted by connecting a pulse oximeter or other measuring device having the instruction part with, for example, a treadmill, bicycle ergometer, or other training machine found in sports gyms, etc., using a cable or wirelessly, so that while the measurement part (measuring device) measures the user's blood oxygen concentration, the instruction part indicates a speed, incline, resistance, etc., for the exercise machine, such as treadmill or bicycle ergometer, and instructs the user to achieve a workload according to the ramping load.

The estimation system proposed by the present invention obtains the blood oxygen concentration (SpO₂) over a range that covers at least a part of 90 to 100%, where the measurement band is preferably 2% or greater, or more preferably 3% or greater, or yet more preferably 4% or greater, or even more preferably 5% or greater, such as 89 to 100%, 92 to 100%, 94 to 100%, 95 to 100%, 96 to 100%, 92 to 99%, 94 to 99%, 95 to 99%, etc. Preferably the estimation system proposed by the present invention allows the measurement range of SpO₂ to be changed according to the subject's age, sex, exercise habits or lack thereof, etc., where its minimal value is preferably 90% or greater, or more preferably 92% or greater, or yet more preferably 94% or greater, or even more preferably 96% or greater.

The estimation system proposed by the present invention measures the SpO₂ over a range that covers at least a part of 90 to 100%, where a preferred minimal value is 92% or greater, or more preferred minimal value is 94% or greater, or yet more preferred minimal value is 96% or greater, so that the burdens on the subject are reduced during measurement. Also, the estimation system proposed by the present invention can be constituted by combining a pulse oximeter or other measuring device capable of measuring the blood oxygen concentration, with a training machine capable of measuring the ramping load, and therefore it can be introduced to sports gyms, etc., for example, because there is no need to use a special measuring device under the guidance of an expert.

### • Method for Determining Inflection Point

Under the present invention, the method for determining an inflection point of the calculated SpO₂/pulse rate is not specifically limited, but it can be the following method, for example.

A method that measures the pulse rate simultaneously with the SpO₂ and, by using the pulse rate as an independent variable and the value obtained by dividing the SpO₂ by the pulse rate (SpO₂/pulse rate) as a dependent variable, and
based on a combination of regression line 1 covering the measurement points from the first measurement point post-optimal exercise intensity up to the nth measurement point (n≥2) and regression line 2 covering the n+1th measurement point to the Nth measurement point (N≥n+2), determines the point of intersection between regression lines 1 and 2 when the sum of the residual sums of squares of regression lines 1 and 2 becomes the smallest, or the nth measurement point, n+1th measurement point, midpoint thereof (n+0.5), etc., when the sum of the residual sums of squares becomes the smallest, as an inflection point.

Since this determination method requires that exercise is continued until an inflection point appears, the measurement is continued until the heart rate reaches preferably 140 beats/min, or more preferably 150 beats/min, or yet more preferably 160 beats/min, or even more preferably 180 beats/min. However, this upper limit is preferably no higher than 90%, or more preferably no higher than 85%, or yet more preferably no higher than 80%, of the expected maximum heart rate (220 - Age) of the subject.

This determination method also allows that, when, pertaining to the measurement points post-optimal exercise intensity up to Mth measurement point, the sum of the residual sums of squares of regression lines 1 and 2 becomes the smallest based on a combination of regression line 1 covering the measurement points from the first measurement point post-optimal exercise intensity up to the mth measurement point and regression line 2 covering the m+1th measurement point to the Mth measurement point, and also when, pertaining to the measurement points up to the M+pth measurement point obtained by continuing the exercise further, the sum of the residual sums of squares becomes the smallest based on a combination of regression line 1 covering the measurement points from the first measurement point up to the mth measurement point and regression line 2' covering the m+1th measurement point to the M+pth measurement point, the point of intersection between regression lines 1 and 2 or 1 and 2' at that time, or the mth measurement point, m+1th measurement point, or midpoint thereof (m+0.5), etc., when the sum of the residual sums of squares becomes the smallest, can be determined as an inflection point and further measurement can be stopped. If the measurement is stopped midway as described above, preferably M is 5 or greater, while preferably p is 2 or greater. Also, the measurement time from the first to the mth, and that from the m+1th to the Mth, are each preferably 20 seconds or longer, or more preferably 30 seconds or longer, or yet more preferably 40 seconds or longer. Additionally, the measurement time from the Mth to the M+pth is preferably 10 seconds or longer, or more preferably 15 seconds or longer, or yet more preferably 20 seconds or longer.

Under the estimation method proposed by the present invention, exercise needs to be performed only within a prescribed range of pulse rates and there is no need to continue exercising until just before one can no longer move, which can minimize the physical burdens on the subject.

Under the method and system for estimating maximal exercise intensity proposed by the present invention, the method for estimating an optimal exercise intensity is not specifically limited; however, as described in Patent Literature 4, for example, preferably the exercise intensity at the first inflection point at which the behavior of SpO₂/pulse rate changes, or exercise intensity at a starting point of decline at which the measured value of blood oxygen concentration starts to show a declining trend, when a ramping load is given to the subject, is estimated as an optimal exercise intensity. While an optimal exercise intensity can be estimated beforehand, preferably it is estimated simultaneously with a maximal exercise intensity because it can lessen the burdens on the subject. For reference, what is disclosed in Patent Literature 4 is incorporated in its entirety in this Specification.
(1) Estimating the exercise intensity at the first inflection point at which the behavior of SpO₂/pulse rate changes, as an optimal exercise intensity.
   In this case, an optimal exercise intensity can be obtained using the same method as the aforementioned method for determining an inflection point, where the inflection point that appears first is estimated as an optimal exercise intensity and the inflection point that appears post this optimal exercise intensity (second inflection point) is estimated as a maximal exercise intensity.
(2) Estimating the exercise intensity at a starting point of decline at which the measured value of blood oxygen concentration starts to show a declining trend, as an optimal exercise intensity.

The anaerobic threshold (AT) is a point at which aerobic exercise switches to anaerobic exercise, and at the AT the oxygen concentration in the body starts to drop due to a lack of oxygen supply needed to continue exercising/produce energy. Since this exercise intensity at a starting point of decline approximates the exercise intensity at the AT, the exercise intensity at a starting point of decline can be estimated as an optimal exercise intensity.

This starting point of decline can be determined by method 2-1 or 2-2 below, for example.

### • Method for Determining Starting Point of Decline 2-1

A method that measures the pulse rate simultaneously with the SpO₂ and determines a starting point of decline based on change in SpO₂ over time,
wherein the method is such that, once the pulse rate first exceeds the target pulse rate, the value of SpO₂ at the time the pulse rate first exceeded the target pulse rate is used as a reference value, and the measurement point of SpO₂ immediately before a region where the measured value of SpO₂ indicated a value lower than the reference value consecutively for 5 seconds or longer is determined as a starting point of decline.

### • Method for Determining Starting Point of Decline 2-2

A method that measures the pulse rate simultaneously with the SpO₂ and determines a starting point of decline based on change in SpO₂ over time,
wherein the method is such that, once the pulse rate first exceeds the target pulse rate, the value of SpO₂ at the time the pulse rate first exceeded the target pulse rate is used as a reference value, and the point of intersection between a straight line connecting the measurement point of the highest value and measurement point of the lowest value, of SpO₂, in a region where the measured value of SpO₂ indicated a value lower than the reference value consecutively for 5 seconds or longer, and an approximate straight line before the region, is determined as a starting point of decline. If there are two or more measurement points giving the highest value or lowest value, one of such values obtained first chronologically is used as the measurement point.

Under these methods 2-1 and 2-2, the target pulse rate can be any value used as a reference for aerobic exercise; for example, a value of target heart rate derived by the Karvonen formula {(220 - Age - Stationary heart rate) x (0.4 to 0.7) + Stationary heart rate}, or a value reflecting a simplified version of it (220 - Age) x 0.5 to 0.7, etc., can be used, or simply a value of around 120 to 130 beats/min can be used.

Under methods 2-1 and 2-2, the number of seconds over which the measured value of SpO₂ indicates a value lower than the reference value (value of SpO₂ at the time the pulse rate first exceeded the target pulse rate) only needs to be 5 seconds or more. The longer these seconds, the lower the possibility becomes of determining a wrong starting point of decline based on measured values deviating from the trend; however, a longer measurement time increases the physical burdens on the subject. Accordingly, the lower limit of these seconds is preferably 8 seconds or more, or more preferably 10 seconds or more, while the upper limit of these seconds is preferably 60 seconds or less, or more preferably 50 seconds or less, or yet more preferably 40 seconds or less.

Also, preferably a judgment is made based on measured values from two or more consecutive multiple measurements to prevent misjudgment caused by a single mismeasurement. Specifically, when the measured values of SpO₂ are obtained as average values over 1 to 30 seconds or so, preferably the value obtained by multiplying the number of seconds over which to calculate a measured value (average value) of SpO₂ by the (number of measurements - 1) is 5 seconds or greater, and, at the same time, two or more consecutive multiple measurement points deliver values lower than the reference value.

### • Training Method

The training method proposed by the present invention is characterized in that exercise is performed using, as a guideline, the maximal exercise intensity estimated by the aforementioned estimation method.

Using the estimation method proposed by the present invention, the exercise intensity at an inflection point at which the behavior of SpO₂/pulse rate changes as the workload increases beyond an optimal exercise intensity can be estimated as a maximal exercise intensity for the subject. Then, because this estimated maximal exercise intensity approximates the exercise intensity at the RCP, individuals who are required to attain exceptionally high levels of motor abilities can improve their body strength efficiently according to a training method whereby exercise is performed using this maximal exercise intensity as a guideline. To be more specific, training is performed at exercise intensities that are inside a range from the optimal exercise intensity to maximal exercise intensity and also correspond to preferably 80 to 95% or so, or more preferably 85 to 95% or so, or yet more preferably 90 to 95% or so, of the maximal exercise intensity.

### • Exercise Instruction Device

The exercise instruction device proposed by the present invention is characterized in that it comprises: a storage means for storing biological information values at the maximal exercise intensity estimated by the estimation method proposed by the present invention; a measurement means capable of measuring the biological information values; and an instruction means for instructing an exercise intensity by comparing the biological information values measured by the measurement means against the biological information values at the maximal exercise intensity.

The exercise instruction device proposed by the present invention can also have, besides the above, a memory or other storage means, a communication means, a display means, a CPU or other computation means, a battery, and so on. Also, the storage means and computation means can be such that their processing takes place at least partially on an external server with which it communicates through its communication part.

The mode of the exercise instruction device proposed by the present invention is not specifically limited, and, for example, the exercise instruction device may be built into a training machine or it may be an external terminal connected to a training machine, such as a smartphone, smart watch, smart glasses, earphone, etc., that is made capable of implementing the aforementioned means by installing an application therein. Preferably, of these, the exercise instruction device is a smart watch, smart glasses, or other wearable terminal.

In the exercise instruction device proposed by the present invention, the biological information values that are measured/stored include, besides the SpO₂ and pulse rate, the blood pressure, lactate concentration (in blood, in sweat), carbon dioxide concentration in exhaled breath, etc., of which one type, or two or more types, may be measured/stored. Preferably, of these, the SpO₂ and pulse rate are measured and stored because of their ease of measurement.

The exercise instruction device proposed by the present invention can, by having the computation means compare the biological information values at the estimated maximal exercise intensity stored by the storage means against the biological information values measured by the measurement means, calculate the difference between the current exercise intensity and the maximal exercise intensity to calculate an exercise intensity suitable for the objective. Here, preferably exercise intensities can be set at, e.g., weak/equal/strong with respect to the maximal exercise intensity which is used as a guideline. This way, exercise intensities suitable for the objective can be selected according to one's physical condition, the number of days until the game, etc., and so on. Then, by exercising at the exercise intensities instructed by the exercise instruction device proposed by the present invention, the user can train himself/herself quite efficiently.

In addition, preferably the exercise instruction device proposed by the present invention can store the biological information values at an optimal exercise intensity and instruct an exercise intensity by comparing the biological information values measured by the measurement means against the biological information values at the optimal exercise intensity. This way, exercise intensities suitable for various objectives can be set according to the effects obtained by exercising at levels between the optimal exercise intensity and the maximal exercise intensity.

Furthermore, preferably the exercise instruction device proposed by the present invention is such that the measurement means is capable of measuring the SpO₂ and pulse rate, the instruction means is capable of instructing a workload based on the information, and the computation means is capable of calculating an inflection point at which the behavior of SpO₂/pulse rate changes as the workload increases beyond an optimal exercise intensity.

Such exercise instruction device can estimate a maximal exercise intensity for the user by virtue of its ability to instruct a workload and calculate an inflection point post-optimal exercise intensity at which the behavior of SpO₂/pulse rate changes. Therefore, assume, for example, that the user has improved his/her physical abilities by exercising continuously for a while at the estimated maximal exercise intensity instructed by the exercise instruction device proposed by the present invention and thus can no longer expect much improvements in his/her physical abilities at this exercise intensity; even in this situation, by obtaining the latest maximal exercise intensity, exercise can be effectuated at this exercise intensity representing a higher load. Here, preferably the exercise instruction device can instruct a workload that will be used as a ramping load, and preferably it can also calculate an optimal exercise intensity from the first inflection point at which the behavior of SpO₂/pulse rate changes as the workload increases.

### Examples

### (Examples)

Workload application method
Equipment used: Ergometer
Load application method: Ramping load method
   Ergometer setting - Crank speed 60 rpm
   The saddle is adjusted so that the subject's knee bends slightly when the pedal is at the lowest point.
   Resting condition - Rest for 2 minutes in a sit-down position (seated on the Ergometer).
   Warm-up condition - 5 minutes at 50 watt
   Workload application condition - Ramping load increment 10 watt/min
   Stopping condition - Load is lifted when one of the following conditions is satisfied:
      1) The subject can no longer carry on sustained exercise at 60 rpm due to fatigue in the lower limbs.
      2) The person in charge of testing determines that the test should be aborted.
      3) The SpO₂ or pulse rate exceeds the set measurement range.
   Unit of load: watt

### (Measurement of SpO₂ and Pulse Rate)

The blood oxygen concentration (SpO₂) and pulse rate were measured using a pulse oximeter (NELLCOR^{™} N-BSJ (Covidien Japan Inc.)).

The SpO₂ and pulse rate were measured at 4-second intervals and averaged every 20 seconds.

Measurement was performed over a SpO₂ range of 96 to 100% and also with the upper limit of pulse rate set to 160 beats/min.

### (Measurement of Exhaled Gas Parameters and Heart Rate)

Measuring equipment: Exhaled gas analyzer AEROMONITOR AE-310SRC (Minato Medical Science Co., Ltd.), heart rate meter POLAR, heart rate sensor H10 N Measuring method: Mixing chamber method
Items measured: Carbon dioxide production (VCO₂: ml/min), oxygen consumption (VO₂: ml/min), minute ventilation (VE), partial pressure of end-tidal oxygen (PETO₂), partial pressure of end-tidal carbon dioxide (PETCO₂), end-tidal oxygen concentration (ETO₂), end-tidal carbon dioxide concentration (ETCO₂), gas exchange rate (R=VCO₂/VO₂)*, ventilatory equivalent for oxygen (VE/VO₂)*, ventilatory equivalent for carbon dioxide (VE/VCO₂)*, heart rate (HR: bpm) * Calculated value
Data acquisition frequency: Once every 20 seconds

Subjects A to E were given workloads according to the described method, and their exhaled gas parameters, heart rate, SpO₂, and pulse rate were measured. For subjects A to D, measurement was performed until their SpO₂ became 96%; for subject E, measurement was performed until the subject could no longer carry on sustained exercise at 60 rpm, i.e., until the subject's maximum exercise intensity was reached.

### • Determination of Respiratory Compensation Point (RCP) According to Golden Standard Method

The values measured every 20 seconds from the start to end of ramping load application were plotted, and RCPs were obtained, according to the conventional golden standard method using exhaled gas parameters, from the point at which the PETCO₂ began a sustained decline post-optimal exercise intensity, from the point at which the VE/VCO₂ began a sustained rise post-optimal exercise intensity, and also from the point at which the VE began increasing faster than the rate of increase in VCO₂ post-optimal exercise intensity.

### • Determination of Inflection Point According to Present Invention

The pulse rate was measured simultaneously with the SpO₂, and
by using the pulse rate as an independent variable and the value obtained by dividing the SpO₂ by the pulse rate as a dependent variable,
the N+1th measurement point when the sum of the residual sums of squares of two regression lines A and B became the smallest based on a combination of regression line A covering the measurement points from the first measurement point up to the Nth measurement point (N≥2) and regression line B covering the N+1th measurement point to the last measurement point, was determined as the first inflection point and an optimal exercise intensity was estimated accordingly.

From the data at the measurement points after the estimated optimal exercise intensity (N+1th to last measurement points), the N+M+2th measurement point when the sum of the residual sums of squares of two regression lines 1 and 2 became the smallest based on a combination of regression line 1 covering the measurement points from the optimal exercise intensity (N+1th) up to the Mth measurement point (M≥2) and regression line 2 covering the N+M+2th measurement point to the last measurement point, was determined as the second inflection point and a maximal exercise intensity was estimated accordingly.

The pulse rates at the maximal exercise intensities estimated by the aforementioned methods are shown in Table 1 along with the pulse rates at the RCPs measured by the golden standard method (GS method) based on exhaled gas analysis.

**[Table 1]**

| Table 1 | Pulse Rates at Maximal Exercise Intensities or RCPs | | | |
|---|---|---|---|---|
| | Present invention | PETCO2 | VE/VCO2 | VE |
| A | 149 | 155 | 150 | 147 |
| B | 153 | 150 | 150 | 146 |
| C | 131 | 131 | 125 | 124 |
| D | 135 | 140 | 135 | 137 |
| E | 140 | 145 | 145 | 137 |
| Average | 142 | 144 | 141 | 138 |
| Standard deviation | 9.3 | 9.3 | 10.8 | 9.3 |
| Standard error | 3.1 | 3.1 | 3.6 | 3.1 |
| r | | 0.911 | 0.926 | 0.917 |

For every subject, the pulse rate at the maximal exercise intensity estimated according to the estimation method proposed by the present invention turned out to be a value very close to the pulse rates at the RCPs measured by the conventional golden standard method (GS method). In other words, it was confirmed that a maximal exercise intensity roughly equivalent to the maximal exercise intensities obtained as RCPs by the GS method can be estimated according to the estimation method proposed by the present invention.

The results of arranging the SpO₂ and pulse rates by bringing the pulse rates at the optimal exercise intensities to position No. 4, are shown in Table 2. In Table 2, the SpO₂ and pulse rates at the maximal exercise intensities are shown in bold.

### [Table 2]

**Table 2**

| | A | | B | | C | | D | | E | |
|---|---|---|---|---|---|---|---|---|---|---|
| | SpO2 | Pulse rate | SpO2 | Pulse rate | SpO2 | Pulse rate | SpO2 | Pulse rate | SpO2 | Pulse rate |
| 1 | 98.0 | 122 | 97.0 | 125 | 97.2 | 110 | 96.8 | 117 | 99.0 | 110 |
| 2 | 98.0 | 122 | 96.0 | 129 | 97.0 | 112 | 97.0 | 116 | 99.0 | 113 |
| 3 | 98.0 | 124 | 96.2 | 131 | 97.0 | 117 | 97.0 | 119 | 99.0 | 116 |
| 4 | 98.0 | 127 | 96.2 | 133 | 96.6 | 116 | 97.0 | 119 | 98.6 | 118 |
| 5 | 98.0 | 129 | 96.0 | 136 | 97.0 | 118 | 97.0 | 122 | 99.0 | 123 |
| 6 | 98.0 | 130 | 96.4 | 139 | 97.0 | 118 | 97.0 | 124 | 98.6 | 123 |
| 7 | 97.6 | 131 | 96.0 | 139 | 97.0 | 120 | 97.0 | 125 | 98.2 | 127 |
| 8 | 97.6 | 133 | 96.0 | 141 | 97.2 | 120 | 96.6 | 128 | 98.8 | 129 |
| 9 | 97.0 | 135 | 96.0 | 144 | 98.0 | 121 | 96.0 | 130 | 99.0 | 131 |
| 10 | 96.8 | 139 | 96.2 | 145 | 98.0 | 123 | 96.4 | 132 | 98.8 | 133 |
| 11 | 97.0 | 142 | 96.2 | 148 | 97.2 | 126 | **96.4** | **135** | 98.0 | 136 |
| 12 | 97.0 | 144 | 96.6 | 148 | 97.0 | 125 | 96.6 | 136 | **98.6** | **140** |
| 13 | 96.2 | 147 | 96.0 | 147 | 97.0 | 125 | 96.8 | 139 | 98.2 | 142 |
| 14 | **96.4** | **149** | 96.0 | 151 | 96.0 | 129 | 96.2 | 141 | 98.8 | 144 |
| 15 | 96.0 | 150 | **96.0** | **153** | **96.2** | **131** | - | - | 98.2 | 147 |
| 16 | 96.2 | 153 | 96.4 | 154 | 96.2 | 133 | - | - | - | - |
| 17 | 96.0 | 156 | 96.4 | 156 | 96.0 | 136 | - | - | - | - |
| 18 | - | - | 96.0 | 157 | 96.4 | 139 | - | - | - | - |

| | | | | | | | | | | |
|---|---|---|---|---|---|---|---|---|---|---|
| Optimal exercise intensities at No. 4, maximal exercise intensities in bold | | | | | | | | | | |

For subjects A to D, maximal exercise intensities could be estimated with the subjects exercising at an SpO₂ of 96% or higher.

Subject E exercised to the individual's maximum exercise intensity, at which point the SpO₂ was a relatively high value of 98.2%. This result shows that, for subject E, a maximal exercise intensity could be estimated in a condition where the subject had reached the limits in terms of leg muscles but still had a reserve cardiorespiratory capacity.

## Claims

1. A method for estimating maximal exercise intensity, **characterized by** comprising:
a step to give a ramping load to a subject to obtain a measured value of blood oxygen concentration (SpO₂) at each different workload over a range that covers at least a part of 90 to 100%, while measuring a pulse rate simultaneously with the SpO₂; and
a step to determine an inflection point at which a behavior of SpO₂/pulse rate changes as the workload increases beyond an optimal exercise intensity;
wherein an exercise intensity at the inflection point is estimated as a maximal exercise intensity for the subject.

2. The method for estimating maximal exercise intensity according to claim 1, wherein the optimal exercise intensity represents the exercise intensity at a first inflection point at which the behavior of SpO₂/pulse rate changes, or exercise intensity at a starting point of decline at which a measured value of blood oxygen concentration starts to show a declining trend.

3. A training method, **characterized in that** exercise is performed using, as a guideline, a maximal exercise intensity estimated by the estimation method according to claim 1 or 2.

4. An exercise instruction device, **characterized by** comprising:
a storage means for storing biological information values at a maximal exercise intensity estimated by the estimation method according to claim 1 or 2;
a measurement means capable of measuring the biological information values;
a computation means for calculating an exercise intensity by comparing the biological information values measured by the measurement means against the biological information values at the maximal exercise intensity; and
an instruction means for instructing the exercise intensity calculated by the computation means.

5. The exercise instruction device according to claim 4, **characterized in that**:
the measurement means is capable of measuring a blood oxygen concentration (SpO₂);
the instruction means is capable of instructing a workload based on information relating to biological information values from the measurement means; and
the computation means is capable of calculating an inflection point post-optimal exercise intensity at which a behavior of SpO₂/pulse rate changes as the workload increases.

6. The exercise instruction device according to claim 4, **characterized by** being a wearable terminal.

7. A system for estimating maximal exercise intensity, **characterized by** comprising:
a measurement part that measures a blood oxygen concentration (SpO₂) over a range that covers at least a part of 90 to 100%, while also measuring a pulse rate;
an instruction part that instructs a workload that will be used as a ramping load; and
a computation part that calculates an inflection point at which a behavior of SpO₂/pulse rate changes as the workload increases beyond an optimal exercise intensity;
wherein an exercise intensity at the inflection point is estimated as a maximal exercise intensity.

8. The system for estimating maximal exercise intensity according to claim 7, wherein the optimal exercise intensity represents the exercise intensity at a first inflection point at which the behavior of SpO₂/pulse rate changes, or exercise intensity at a starting point of decline at which a measured value of blood oxygen concentration starts to show a declining trend.
